# EUROPEAN PATENT SPECIFICATION

(11) **EP 0 573 060 B1**
(45) Date of publication and mention of the grant of the patent: **09.02.2000**
(21) Application number: 93109012.0
(22) Date of filing: 03.06.1993
(51) Int. Cl.: G01N 33/497, G01N 27/66, G01N 27/62, A61B 5/083, G01N 33/00

(54) **Expired air examination device and method for clinical purpose**
Vorrichtung und Verfahren zur Untersuchung ausgeatmeter Luft für klinische Zwecke
Appareil et méthode pour examiner de l'air expicée à des fins cliniques

(30) Priority: 03.06.1992 JP 17028792; 30.06.1992 JP 19769992
(43) Date of publication of application: 08.12.1993
(73) Proprietor: Ueda, Hideo, Minoh-shi, Osaka (JP); KABUSHIKI KAISHA KYOTO DAIICHI KAGAKU, Kyoto-shi Kyoto 601 (JP)
(72) Inventor: Ueda, Hideo, Minoh-shi, Osaka (JP); Hiromoto, Mitsuo, Uji-shi, Kyoto (JP)
(74) Representative: Patentanwälte Hauck, Graalfs, Wehnert, Döring, Siemons

(56) References cited:
- US-A- 3 883 739
- US-A- 4 114 422
- US-A- 4 413 185
- US-A- 4 735 777
- US-A- 4 772 559
- US-A- 4 994 096

## Description

### BACKGROUND OF THE INVENTION

Various clinical examinations to be carried out for diagnosis, treatment or prevention of diseases are essential in the contemporary medical science. Particularly, clinical biochemical examinations for analyzing particular compounds occupy an important position in the clinical medical science. Furthermore, following development of the basic medical science and advancement of analyzing techniques, attempts have been made on enlargement of the extent of samples to be examined and items to be measured, improvement of measurement accuracy and automatization of measuring techniques at various measuring stages, such as an abbreviated rapid measurement including urgent or bedside measurement, or systematized measurements carried out in large-scaled hospitals.

Samples to be used in the clinical biochemical examinations at the present time are generally blood and urine. Blood provides quite ample information and covers several hundreds of items to be measured. But, collecting blood causes subjects or patients a physical pain with loss of their important blood, and repeated collection of blood and a continuous measurement thereof impose a heavy burden on the subjects or patients. In addition, analyzing the blood samples is delt exclusively or centrally by a blood analysis center (firms specialized in analyzing blood) to occupy much time from collecting blood to the analysis, leading to larger errors in measurement of certain contents of blood which easily gasified or denatured. Hence, such unstable components are exceptionally measured only at large-scaled hospitals provided with special analyzing apparatuses. Meanwhile, urine is discharge relatively readily and amply collectable and broadly used in screening, but has a problem that it provides less information than blood does. Also, the examinations with urine causes the subjects or patients a mental pain as from a feeling of shame and do not enable a continuous measurement, and urine is hard to be collected at all times.

Other clinical analyzing techniques in bloodless and noninvasive type than the urine analyzing technique, for example, those using as samples such body fluid other than blood as exudate from skin (lymph), sweat, saliva or the like, or percutaneously measuring blood gas with a blood gas sensor, have been hitherto developed. But, these techniques have not been broadly put into practical use due to the facts that merely less amount of samples are obtainable and measurable items are limited. Hence, the main stream of development of the clinical biochemical examination techniques is now directed to those which do not cause the subjects to pain in collecting blood and which measure a larger extent of items with less trace amounts of blood samples.

In any cases, the conventional clinical biochemical examinations require much labor and specific techniques for collecting, transporting, preserving, separating and analyzing samples such as blood and urine, so that they have the problems in examination accuracy, rapidity and labor-saving and cause the subjects (patients) an unnecessary pain. Solution of the problems will be a great boon not only to the patients but also to workers engaging in medical treatments.

It is said that doctors having much experiences in medical treatments judge names of diseases from special smells emitted by patients and use the smells as important information in inspection, and that expired air of patients with diabetes does smell like acetone or of a particular sweet smell and those of patients with a liver sickness or leukaemia of ammonia or sulfide. But, these information is obtainable based on the doctors experiences, may be judged differently by specific doctors and cannot be objectively shown by data. Also, the clinical biochemical examinations using subjects' expired air as samples has almost not been put into practical use and basic studies thereof have not been advanced.

This is firstly because there is a prejudice in the art that expired air should not be usable as samples for the clinical biochemical examination, and secondly because gases of the expired air to be detected are quite low in concentration (ppb or ppm at the most) so that they could be measurable only by a combination of a concentrating device of the trace amounts of gas component and a large-scaled high-sensitivity gas detecting device. Hence, measurement of the samples can be carried out only in a laboratory provided with the special instruments and equipments operable by skilled operators, and there are heard only few clinical reports in this kind of measurement. Such clinical reports the present inventor knows are (1) Dubowski, K,M, Breath Analysis as a Technique in Clinical Chemistry, Clin .Chem., 20.966-972, 1974, (2) Manolis,A., The Diagnostic Potential of Breath Analysis, Clin.Chem., 29. 5-15, 1983, and (3) Phillips, M., Breath Tests in Medician, Scientific American July.1992. These reports disclose measurement and observation data only.

Expired air is intermittently breathed out by human (or animals) during their lives are maintained, and it is readily collectable, without causing subjects a physical or mental pain, particularly from infants, patients with serious illness or damages in consciousness by use of a specially designed instrument. Also, since trace amounts of volatile component of mixed venous blood flowing through alveolar blood capillary is moved into expired air by gas exchange, it is inferred that expired air and blood have correlation with respect to the volatile component. Furthermore, examinations with expired air enables differential measurement of the volatile component which measurement the blood analysis could not cover. Thus, expired air is an ideal sample for the clinical biochemical examination.

Expired air when collected in vessels occupies much spaces for preservation and transportation and some gas components subject to examination are unstable, so that they are not well subjected to the analyzing course as of blood, i.e., to be first transported to the analysis center and analyzed by the large-scaled apparatuses. That is, the clinical biochemical examination using expired air as sample is necessarily to be carried out on a face to face basis, and expired air may be effectively used only in such manner of measurement that a measuring operator (an analyzing person) directly faces to a subject (a patient), such as a bedside examination, a pre-hospital examination in an ambulance, screening upon medical examination and monitoring of states of patients (continuous monitoring).

Hence, the foregoing apparatus comprising the combination of the concentrating device and the large-scaled high sensitivity gas detecting device and used in some local laboratories is not practically proper for the clinical biochemical examination using expired air as samples which examination does just require an examination device which is small-sized, portable, of high sensitivity, simply operable and superior in safety and rapidity in measurement. Reliability of provided data and economization in use of such examination device are also required. Furthermore, since it is possible that moisture of expired air in a container makes dew on a wall of the container to dissolve and adsorb trace amounts of gas components, such type of examination device is preferable that expired air from a subject (patient) is sucked directly into the device to be subjected to measurement.

A expired air examination device according to the preamble of claim 1 is known from US-A-4114422. An apparatus for detection of vapours from low volatil compounds using an electron capture detector is disclosed in US-A-3883739.

### SUMMARY OF THE INVENTION

An object of the invention is to provide a novel bloodless and noninvasive clinical examination device and more particularly to a portable type expired air examination device which analyzes expired air samples of subjects to determine concentration of trace amounts of gas components of the expired air samples. Another object of the invention is to provide an expired air examination device which is of high sensitivity in operation, readily operable, applicable to infants, aged men and women and unconscious subjects and provides safety, reliability of obtainable data and economization in use. A further object of the present invention is to provide an expired air examination device and an expired air examining method which enable rapid measurements for urgent cases and any cases requiring to examine a number of subjects as quickly as possible.

The invention is defined in claim 1. Preferred embodiments are defined in the subclaims.

### BRIEF DESCRIPTION OF THE DRAWINGS

Fig. 1 is a block diagram showing an expired air examination device for clinical purpose according to the present invention.
Figs. 2, 3 and 4 are block diagrams showing modified examples of the carrier gas feeding part.

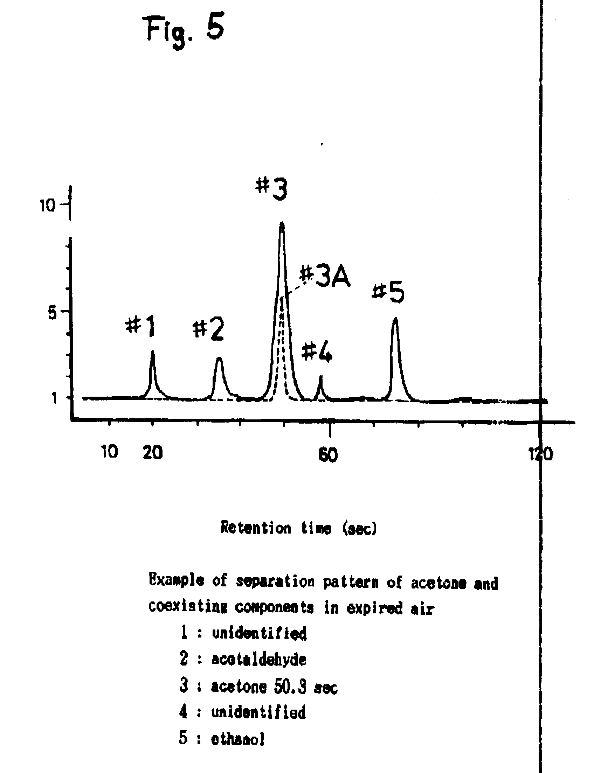

Fig. 5 is a gas chromatogram of ketone body (acetone) measured by the device according to the present invention, wherein the broken line shows the measurement using a standard substance as a sample and the solid line that using expired air of diabetics.

### DETAILED DESCRIPTION OF THE INVENTION

The inventor has zealously studied under the foregoing circumstances in order to develop a practical bloodless and noninvasive clinical examination device and method using expired air as sample and achieved the present invention which will be detailed hereunder.

The expired air examination device according to the present invention employs expired air of human (or animals) as samples, separates and measures trace amounts of chemical substances of the expired air to obtain various data of clinical biochemical information. The reference "bloodless" means an examination method using as samples the specimens other than blood, and the reference "noninvasive" means no-breaking and is directed to an examination method wherein samples are collected without hurting human bodies.

The expired air examination device according to the present invention generally comprises an expired air injecting part, a carrier gas feeding part; a detecting part and an arithmetic processing part. The detecting part does specially employ a particular construction so as to have high sensitivity and be small-sized as required.

In detail, analysis of gases generally adopts gas chromatography and uses various detectors depending on sorts of the gases and specific purposes of the analysis. The examination device according to the present invention adopts a separator column, particularly, a capillary column for providing measurement of a plurality of items and a rapid measurement. And the invention uses as the detector a PID (Photo Ionization Detector), wherein each gas component of expired air to be detected is applied with ultraviolet radiation to be ionized so that measurement signals are output corresponding to amounts of ionization of the gas component.

This type of detector used in the present invention does not have burning of hydrogen gas as in FID (Flame Ionization Detector) or FPD (Flame Photometric Detector) and is thereby safe and small-sized, and also of high sensitivity and high accuracy in comparison with FID and FPD, and further has the advantage that air which is cheap can be used as carrier gas. Also, APIMS (Atmospheric Pressure Ionization Mass Spectometry) which is of quite high sensitivity and high accuracy is quite large in size and not applicable to the present invention. Furthermore, such a detector having high sensitivity and high accuracy as controlled-potential electrolysis gas sensor is to be selectively used, i.e., to be limited of use to gases such as carbon monoxide or hydrogen compounds. Hence, such detector is also not applicable to the present invention. To be noted is that PID does not use radiation.

The expired air sucking part is adapted to feed expired air (expired gas) to the sample weighing part and comprises an expired air collector such as a mouthpiece or a mask for collecting expired air and a collecting tube (a sampling probe) mounting the expired air collector at one end. The expired air sucking part, particularly, the collecting tube (sampling probe) is preferably to be heated at its inner surface to human body temperature or higher temperatures, for example, 36 to 100°C, more preferably 40 to 50°C. This prevents that moisture of the expired air condenses on and sticks to an inner wall of the collecting tube to dissolve and adsorb an aimed gas component. For heating the collecting tube, a heating element may be disposed circumferentially of or inside the collecting tube, or the tube may be made of a material having itself heat build-up and sheathed with insulating material. Also, the collecting tube may include a temperature adjuster mechanism. The expired air collector may be disposable type to be sanitary.

Such feature may be adopted that expired air is collected first in a separate expired-air collecting means such as a large injector or a balloon (but not directly blown into the examination device) and then fed into the device through the collecting tube. Alternatively, expired air collected at a predetermined amount by a syringe may be injected from an expired air sample injecting part formed at the upper stream side of the column. In these cases, the expired air collecting means, i.e., the injector, balloon and syringe requires to be kept at specific temperatures or heated so that inside of the expired air collecting means can be kept at temperatures higher than human body temperature.

The sample weighing part collects and holds as an expired air sample a predetermined amount of portion of expired air fed from the expired air sucking part. The collection of the predetermined amount of air portion may be carried out by patient's positive blowing to push the expired air or preferably by use of a sampling pump to take in or press the expired air. Use of the sampling pump has advantages that expired air can be collected readily from infants or unconscious patients and contamination (carry-over) on the inner wall of the collecting tube due to a previous subject's expired air can be purged and removed by taking in atmosphere or a subsequent patient's expired air.

The sample weighing part may be so constituted that a carrier gas passage is provided partially with two sets of valves to form therebetween a weighing chamber whose front end connects with an end of the collecting tube through a valve. Also, the sample weighing part may use a known six-way valve (injection valve) or be so constructed that an end of the collecting tube is connected to a cylinder serving as the weighing chamber to feed into the carrier gas passage an expired air sample collected in the weighing chamber. A predetermined amount of expired air sample depends upon capacity of the detecting part and may be about 0.05 to 5.0 ml, more preferably 0.1 to 0.8 ml. The sample weighing part, particularly, the weighing chamber needs to be kept at a constant temperature higher than human body temperature (for example, the same temperatures as the collecting tube) to prevent sticking of moisture and keep constant the mass of the expired air samples.

The carrier gas feeding part feeds a carrier gas which sends the expired air samples to the separation column. A source of the carrier gas is preferably a small-sized gas cylinder in consideration of the portability of the examination device according to the invention. In case that the examination device is installed at a fixed position for use, a larger gas cylinder is usable for the carrier gas source. Also, since the invention does not use hydrogen flame, it employs as the carrier gas a purified air which is cheap ambient air not the air packed in a cylinder may be used to be fed by a compression pump. The air cylinder, in turn, the vessel itself, is heavy and requires a pressure adjuster, so that the examination device when houses therein the air cylinder is deteriorated in portability. The gas cylinder has further such problems that it is expensive and takes much labor and cost through frequent replacement due to a smaller air content when the air cylinder is designed to be small-sized. By contrary, when ambient air existing innumerably is made use of for the carrier gas, it does not take labor and cost as above and enables the examination device to be further small-sized. In this case, an air filter including adsorbent or the like is provided for purifying taken ambient air to eliminate effects of trace amounts of gas content of the taken ambient air.

In detail, ambient air, i.e., atmosphere existing innumerously is purified on-machine by passing the air through an adsorbing filter and the purified air is given pressure by the compression pump and used as carrier gas. The adsorbing filter is provided for removing particulates (dust) contained in taken atmosphere or contaminant (coexistent) molecules which hinder the aimed examination. The adsorbing filter may comprise adsorbent such as activated charcoal or zeolitic products, a filter sheet of fiber products or the like, high voltage adsorption, or a decomposition means such as an ultraviolet lamp or the like, or any combination of these elements. Also, the compression pump may be a small-sized electric pump which enables compression of 0.5 to 1.0 Kg/cm². Hence, power consumption by the pump together with the detector is small and a battery suffices for a power source. The carrier gas feeding part may further incorporate such instruments as a pressure gauge which monitors stability of the compression pump that pressurizing state is stable or not, a needle valve and a flow meter which control flow of the carrier gas (purified air) to be fed to the separation column, a three-way solenoid valve which releases to atmosphere the carrier gas when not to be flowed to the column. Also, a compressed air reservoir may be provided at the lower stream side of the compression pump.

The detector ionizes oxygen gas to form ozone gas. Although concentration of the ozone gas is about 10 to 50 ppm, formation of ozone is undesirable since the expired air examination is carried out usually in hospitals. (To be noted is that since the ozone gas itself is not detected by the detector, it does not effect on measured values in the examination.) Thus, it is preferable to additionally provide at the lower stream side of the detector an ozone treatment tank which adsorbs the ozone gas or reduces the ozone gas to oxygen gas by use of ultraviolet of any wavelength. Alternatively, a part or all of the waste carrier gas may be mixed, in a mixing tank, with a first purified air (which passed through a first adsorbing filter) and then passed through a second adsorbing filter to be a second purified air which is then given pressure by a compression pump and is used as a carrier gas. In the mixing tank, ozone gas contained in the waste carrier gas oxidizes a sensible content which remains in the first purified air and is a cause of noises sensed by the detector. The first purified air is thereafter passed through the second adsorbing filter made of activated charcoal or zeolitic products, thereby providing highly purified air. Since moisture of the air does not effect on the measurement, dehumidification is not so important. Dehumidifying agent may be included partially in the adsorbing filter when required.

Furthermore, the detecting part comprises a column for separating an aimed gas component contained in an expired air, and a detector. The column employs a capillary column but may alternatively use a packed column depending on sorts of gases to be detected. Fillers for the packed column and liquid layers of the capillary column are selected, corresponding to specific gases to be detected, from various kinds of fillers and liquid layers hitherto known in consideration of such conditions required for the examination as measurement accuracy, reproducibility and rapidity. In this case, when only one kind of gas is detected, a corresponding filler most suitable for determining the gas may be selected. However, certain items (diseases) subject to examination may require measurement of a plurality of gases to be detected, and a plurality of gases may be measured for examining a plurality of items at a time for labor-saving purpose. In such case, when there is found no ideal or suitable filler, such special features may be required that a plurality of columns are used in parallel or a plurality of detectors are employed. Also, the separation column is required to be kept at a constant temperature for providing reproducibility. It is preferable to keep the column at a temperature as lower as possible, for example, room temperature (20°C) to 50°C to prevent decomposition or deterioration of gases to be detected.

The photo ionization detector (PID) is most suitable for the detector of the invention now. The PID utilizes the phenomenon that an aimed gas component is applied with ultraviolet radiation having energy larger than ionization potential of the gas, thereby causing ionization. An amount of ionization of the gas is converted to ionizing current with electrodes to be output, so that concentration of detected gas components is determined from magnitude of ionizing current.

A principal portion of the arithmetic processing part is a microcomputer which controls an operating program for the whole of the expired air examination device as receives measurement signals output from the detector to process the signals, computes concentration of an aimed gas component by use of a previously memorized working curve, stores the computed concentration as a clinical examination data or outputs the data on an indicating device (display) or a recording device (printer), or receives input signals from a keyboard.

Use of the examination device constructed as above according to the present invention is as follows.
(1) The sample collector is first put to or on patient's mouth or face. The measurement start button on the keyboard is depressed and simultaneously the patient is caused to blow breath for few seconds. (When a subject (patient) stops breathing about 10 to 20 sec before blowing breath through the collector and a very small amount of expired air just blown is discarded, remainder can be regarded as alveolar air component equilibrating with mixed venous blood gas partial pressure, thereby enabling measurement with excellent reproducibility.
(2) The expired air is sucked through the collecting tube and a part of the air is collected in the weighing chamber and fed into the separation column by carrier gas.
(3) Trace amounts of an aimed gas component of the expired air separated in the column is ionized in the detector and an amount of ionization of the gas is detected with high sensitivity.
(4) Output from the detector is arithmetically processed and concentration of the aimed gas component is computed on the basis of a previously memorized working curve and stored as a clinical examination data or output on the outputting device. Each measurement ends in dozens of seconds or several minutes depending upon kinds of gases to be detected, so that concentration of a single or a plurality of specific gas component contained in the expired air can be measured rapidly and accurately.

Next, an examination of expired air by use of the expired air examination device according to the present invention will be detailed, exemplifying examinations for diabetes and hyperammonemia in comparison with the conventional analysis with blood and urine.

Diabetes can be examined by screening with urine (urine sugar values), precision examinations with blood (blood sugar, glucose tolerance test, HbAlc, Saccharified Hb test, etc) and measurement of ketone body in blood. (Ketone body is a generic name of the three components, acetone, acetoacetic acid and 3-hydroxybutyric acid.) Diabetics are heard to have increase of ketone body in blood, particularly, 3-hydroxybutyric acid (3-OHBA). Ketone body in blood or urine (screening) is recently measured by nitroprusside test which test in fact cannot accurately measure 3-hydroxybutyric acid. A commercially available product (KETOLEX made by Sanwa Chemical Co.,Ltd.) which makes use of colorimetry using 3-hydroxybutyrate dehydrogenase enables measurement of 3-hydroxybutyric acid, but it has problems of high cost to use and that the reagent cannot be preserved for a long term. Also, concentration in urine does not well correlate with that in blood and the screening has a separate problem, so that only the blood analysis is relied on.

The ketone body has in vivo (in blood) relationship in equilibrium and decomposition with respect to acetoacetic acid (AcAc) as represented in the following formula. $\text{Ac ← AcAc ⇆ 3-OHBA}$The ketone body appears or is discharged, mainly in the form of acetone (Ac), in the expired air through air vesicles, and Ac, AcAc and 3-OHBA are measured in amounts correlating with concentration in blood. By use of the examination device according to the present invention, ketone body (particularly, acetone readily appearing in expired air) can be measured in a quite short time without causing subjects any pain. Hence, the examination device can be applied to screening of diabetes, fasting or malnutrition and also to precision examination and monitoring.

In case of liver dysfunction, particularly, of newborn infants and uremia, ammonia shows high toxicity and 2 mg/dl or more of ammonia causes the patients to have intoxation with coma, and patients with serious liver disorder to have alienation or clouding of consciousness. Ammonia in blood has been hitherto measured by colorimetric analysis with ammonia separated through various techniques or applied with enzyme. But, the methods have problems that they require special analysis instruments and advanced techniques for cooling blood after collected or deproteinization or take much labor, and the tests are hindered by inhibitor substances. Furthermore, ammonia in blood is generally in trace amount and increases after blood is collected and left to stand. Hence, ammonia in blood is to be measured as rapidly as possible by use of a measuring method of high sensitivity. The conventional methods do not fully suffice the purpose and have problems in measurement accuracy.

By contrary, the examination device according to the present invention can rapidly measure concentration of ammonia correlating with ammonia in blood by merely fractionally measuring amounts of ammonia in the expired air without causing patients any pain.

The expired air examination device according to the invention can make the following analytical measurements of most of gas components contained in blood in addition to the foregoing measurement of ketone body and ammonia. In detail, the examination device can carry out rapid measurement of plural components of lower fatty acid serving as an index of liver function test (hepatic insufficiency), simultaneous rapid measurement of ammonia and amine serving as an index of hyperammonemia test, simultaneous rapid measurement of hydrogen sulfide and mercaptan caused from liver diseases (hepatic insufficiency) or oral surgery disorder (ozostomia), simultaneous rapid measurement of lower saturated hydrocarbon (ethane, propane, butane, pentane, etc) serving as an index of existence or non-existence of formation of lipoperoxide (index factor of carcinogenesis), and rapid measurement for monitoring states of intoxation due to chemical substances (intoxation from CO, alcohol or other volatile toxic chemical substances) and grasping pre-treatment conditions.

The examination device enables measurement of phenylacetic acid caused from phenylketonuria which measurement is hitherto carried out by urine analysis, methane and hydrogen (caused from alimentary canal passing-disturbance or intestinal abnormal fermentation) not measurable with blood and urine, and lower hydrocarbon such as ethane and pentane (caused from exposure of organic solvent, lipoperoxide). Sulfuric malodrous components such as hydrogen sulfide and mercaptan sometimes cannot be judged from which they are caused visceral affection or oral surgical diseases. In this case, since mouthwashing with clean water or aqueous solution of hydrogen peroxide or removal of coating of tongue can eliminate influence from ozostomia, expired air may be measured before and after mouthwashing or the like to detect specific gases from the causes.

Furthermore, in case of visceral disorders, specific gas components do not necessarily just correspond to respective disorders, similarly to the examinations with blood and urine. For the screening, a certain one kind of gas may be measured as in the above example to infer degree of a corresponding disease. Also, liquid layers of the column and measurement programs may be set to enaole detection a plurality of gas components so as to carry out measurement of a plurality of disease items or measurement of a single item of disease with high accuracy. Hereunder exemplified are groups of aimed gases in measurement of a plurality of disease items.
(a) Diseases of adult people: screening
   - acetonediabetes
   - ammonialiver, kidney
   - methyl mercaptanliver, kidney
   - acetaldehydeliver, kidney
   - pentaneformation of lipoperoxide
(b) Newborn infants, infants
   - ammoniahyperammonemia (inborn error of metabolism)
   - phenylacetic acidphenylketonuria (inborn error of metabolism)
   - isovaleric acidisovalericacidemia (inborn error of metabolism)
   - methyl mercaptanliver, kidney

The examination device of the invention, particularly, those provided with a sampling pump is adapted to automatically make operation by itself after subjects merely blow their breath in the device. Hence, the examination can be readily carried out, irrespective of ages, sex, states of diseases of patient, for any subjects such as aged men and women, newborn infants, infants, unconscious patients or serious marasmic patients. Furthermore, since the examination device is small-sized and readily operable, it can be quite broadly applicable to be used for screening and diagnosis of metabolic or toxic diseases, tolerance test, monitoring of a course of diseases and as a testing instrument for pre-hospital treatment as in an ambulance.

### EMBODIMENTS

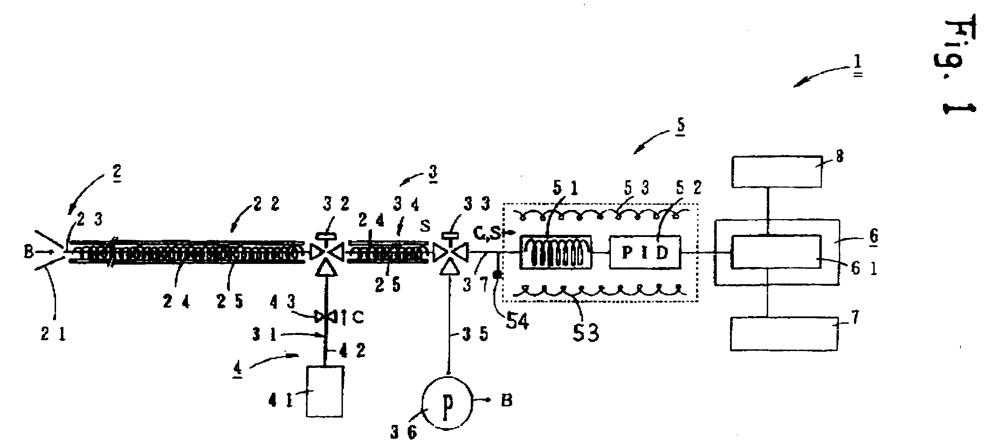

Next, the invention will be detailed with referring to the examples shown in the attached drawings. To be note is that the present invention should not at all be limited to the features shown in the drawings. Fig. 1 is a block diagram showing an expired air examination device 1 according to the present invention. The examination device 1 comprises an expired air sucking part 2, a sample weighing part 3, a carrier gas feeding part 4, a detecting part 5, an arithmetic processing part 6, a keyboard 7 serving as an input device and a printer 8 serving as an output device.

The expired air sucking part 2 comprises an expired air collecting mask 21 serving as an expired air collector and a collecting tube 22 mounting the mask 21 at the utmost end. The collecting tube 22 comprises a Teflon pipe 23 (1 - 5 mm in inner diameter and about 1 m in length) mounting thereon a heater 24 and sheathed with a heat insulating material 25, so that the pipe 23 is heated of the inside to prevent moisture of expired air from sticking on the inner surface of the pipe 23. The heating is carried out at a temperature of 36 to 100°C, for example, 40°C adjusted by a controller. A mouthpiece may be usable instead of the expired air collecting mask 21.

The sample weighing part 3 has a carrier gas passage 31 provided partially with two sets of three-way solenoid valve 32, 33 forming therebetween a weighing chamber 34 to the front end of which an end of the collecting tube 22 is connected through a solenoid valve 32. The weighing chamber 34 is constructed similarly with the collecting tube 22 by use of the same materials, so that the weighing chamber 34 is similarly heated at about 40°C. A capacity of the weighing chamber 34 is about 0.5 ml. A suction pump 36 is connected to the rear solenoid valve 33 through an exhaust tube 35. Also, a sample injection tube 37 connects the solenoid valve 33 with a column 51, so that the carrier gas passage 31 comprises a carrier gas feeding pipe 42, the weighing chamber and the sample injection tube 37. In case that the suction pump 36 is omitted, a patient (subject) may blow through the collecting tube to force his or her expired air.

In case that a predetermined amount of expired air is collected in a syringe in place of the subjects' direct blowing breath in the collecting tube (including the case using the balloon or the large-sized injector), such a modified feature may be used that instead of the expired air sucking part 2, an expired air sample injecting part (inlet) 54 is formed at the upper stream side of the column as shown in Fig. 1, through which part 54 a weighed expired air sample is fed into the column.

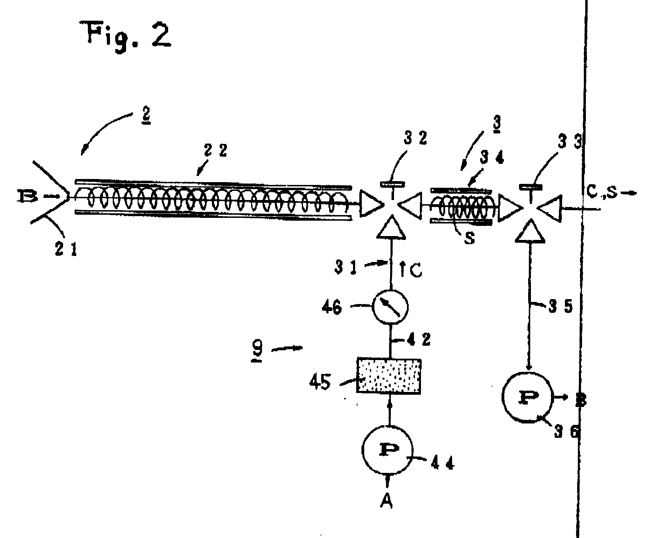

The carrier gas feeding part 4 comprises a small-sized air cylinder 41 containing purified air and the carrier gas feeding pipe 42 connected to the solenoid valve 32. Reference numeral 43 designates a three-way solenoid valve. A carrier gas feeding part 9 shown in Fig. 2 is a modified example wherein without use of the air cylinder 41, ambient air surrounding the device is given pressure by a compression pump to be fed as a carrier gas. In this case, to eliminate influences of trace amounts of gas component in the taken ambient air, an air filter 45 having an adsorbent layer is required to be used. Reference numeral 46 designates a flow meter.

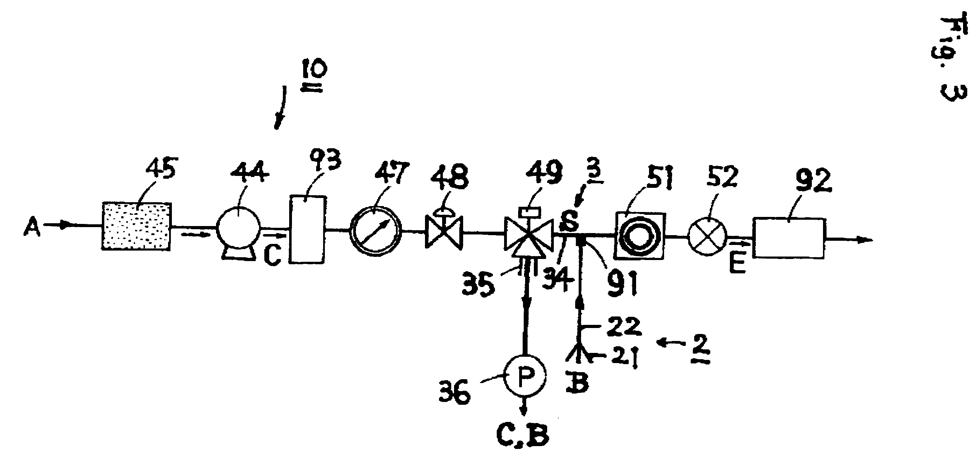

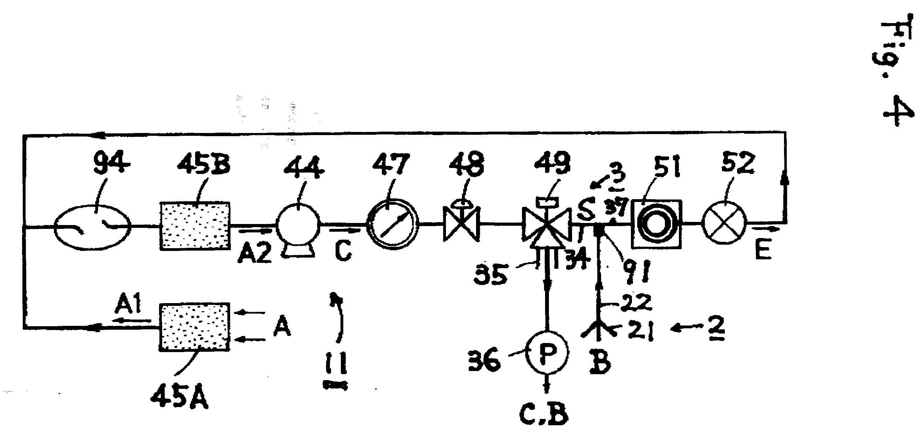

Figs. 3 and 4 are block diagrams showing further modified examples of the carrier gas feeding part using the compression pump. In a carrier gas feeding part 10 shown in Fig. 3, air A taken from atmosphere or ambient air is passed through the air filter 45 to be removed of particulates (dust) or contaminant coexisting molecules and pressurized by the compression pump 44 to be a carrier gas C and go through a flow adjuster means such as a pressure gauge 47 and a needle valve 48 to a three-way solenoid valve 49. The carrier gas C when fed into a column 51 through the three-way solenoid valve 49 carries an expired air sample S stored in the weighing chamber 34 into the column. Aimed gas components contained in the sample are separated orderly in the column and moved into a detector 52, so that the detector 52 outputs measurement signals to the arithmetic processing part (not shown in Fig. 3). The carrier gas C when not fed into the column may be partially leaked through the exhaust tube 35 connected to the solenoid valve 49. 91 is a three-way solenoid valve or an injection valve in Fig. 3. Furthermore, an ozone mixing tank 92 may be provided at the lower stream side of the detector 52 for removing ozone gas contained in the waste carrier gas. 93 is a compressed air reservoir which when mounted enables working intervals of the compression pump 44 to be made longer.

In a carrier gas feeding part 11 shown in Fig. 4, air A taken from atmosphere or ambient air is passed through a first air filter 45A to be removed of particulates (dust) or contaminant coexisting molecules and become a first purified air A1 to be moved into a mixing tank 94. In the mixing tank 94, impurities remaining in the purified air A1 is subjected to oxidization by ozone gas contained in the waste carrier gas E (described later) and further removed at a second air filter 45B to cause the first air A1 to be a second purified air A2 which is then pressurized by the compression pump 44 to become a carrier gas C. Other features in this case are the same as that shown in Fig. 3.

The detector 5 comprises a column 51 for separating aimed gas components contained in the expired air and a detector 52. The column 51 employs a capillary column and the detector 52 a photo ionization detector (PID). The PID applies to the aimed gas components ultraviolet having energy larger than ionization potential of the aimed gases to ionize the gases, convert an amount of ionization of the gases to ionizing current by use of electrodes and output the ionizing current. The detector is quite small-sized but has high accuracy and high sensitivity. The column 51 and detector 52 are kept at constant temperature about 40°C by a heater 53. A liquid layer for the column is selected from known liquid layers to allow specific components of aimed gas, for example, ketone body to have retention time of several dozens of seconds to several minutes.

A principal portion of the arithmetic processing part 6 is a microcomputer 61 which controls an operating program for the whole of the expired air examination device as receives measurement signals output from the detector 52 to process the signals, computes concentration of an aimed gas component by use of a previously memorized working curve, outputs the computed concentration as a clinical examination data on a printer, or receives input signals from a keyboard to output operation commands for specific parts.

In use of the examination device, a main switch on the keyboard 7 is first turned on to have a constant temperature at the specific parts. The expired air collecting mask 21 is fit on the patient's face and the measurement start button on the keyboard 7 is depressed. Expired air B is exhausted outside by the suction pump 36 while a part of the air B is filled in the weighing chamber 34 to be collected as a predetermined amount of expired air sample S by closing the solenoid valves 32 and 33. A subject's blowing expired air is about 5 sec. The expired air sample S is fed into the column 51 by the carrier gas C sent from the air cylinder 41 or the like and separated and fractionated depending on specific retention time of the aimed gases, and ionized orderly in the detector 52, so that amounts of ionization of the gases are converted to electric signals to be output. The electric signals are processed by the arithmetic processing part 6 so that concentration of aimed gas components contained in the expired air (for example, ketone body) are determined by use of previously memorized working curve. Measurement ends about 2.5 min after the expired air of the subject is sucked into the device.

Fig. 6 is a gas chromatogram in measurement of acetone representing ketone body and serving as an examination index of diabetes, malnutrition and fasting. Measurement was carried out with column temperature 45°C and carrier gas (purified air) flow 7.0 ml/min. The broken line in Fig. 6 shows a result of measurement of a standard gas containing acetone 5 ppm (nitrogen balance) and acetone was detected with retention time 50.5 sec (peak #3A). The solid line shows a result of measurement of an expired air of a visitor patient with diabetes (not treated for insulin) and an acute peak (peak #3) was recorded at a position corresponding to retention time 50.3 sec. It is determined from this that the expired air of the patient contains about 8 ppm of acetone. The other peaks show specific components contained in the expired air, peak #2 being identified as acetaldehyde and peak #5 ethanol. Peaks #1 and 4 were unidentified. As seen from the above, ketone body (acetone) serving as an index component can be measured in a short time without having any hindering by other components contained in the expired air.

### EFFECTS OF THE INVENTION

As clearly seen from the above, the present invention relates to an expired air examination device and method for clinical purpose which analyzes an expired air sample of subjects to measure aimed trance amounts of gases contained in the expired air sample. An expired air sample fed from the expired air sucking part is sent through a column to a detector which ionizes trace amounts of aimed gas components contained in the expired air by applying ultraviolet or radiation to the aimed gas components so as to detect such gas components, thereby measuring concentration of the gas components. Hence, the invention has the following characteristics.
(1) The invention uses as sample the expired air which is bloodless and noninvasive and does not cause a patient a pain, a feeling of fear or pressure. Hence, a burden imposed on the patient in relation to repeated measurement of tolerance test and a continuous observation can be completely eliminated.
(2) Operation of the device is merely causing the subjects to blow their breath through the expired air collector. Hence, an operator does not need to have a special training for use the device. Also, the device provided with a sampling pump can readily collect an expired air sample from infants or patients with serious illness or unconsciousness.
(3) Since the expired air collecting tube (sampling probe) is heated at its inside wall, there is no loss of trace amounts of aimed components contained in the expired air and measured values with accuracy can be obtained with reproducibility.
(4) Since a detector which needs no burning of hydrogen and enables measurement with high sensitivity in a short time is used, the examination device can be made small-sized, readily operable and enable rapidity of measurement with a quite low cost for the examination.
(5) Since the device can be small-sized, it can be used as a bedside instrument or mounted on an ambulance without being limited of sites for use. Also, measurement results can be obtained and recorded quickly (in 2 - 3 min), so that it provides a wide range of clinical application, for example, the measurement results can be instantly made use of as a clinical data.
(6) The device uses ambient air as carrier gas by purifying the ambient air on-machine and is therefore small-sized and light-weight and reduces the running costs.
(7) The waste carrier gas is passed through the ozone treatment tank and mixed with sucked ambient air to thereby treat ozone gas, thereby causing no contamination on the environments.
(8) Adoption of a plurality of columns enables measurement of a plurality of aimed gases (each having different retention time) in a short time to improve rapidity of the examination.
(9) The examination device does not at all cause subjects a pain and provides examination results instantly, so that diseases can be located at an early stage.
(10) The invention can provide measurement results more readily and rapidly in comparison with examinations with blood and also can provide much information in comparison with examinations with urine or other bloodless and noninvasive clinical examination method, so that the invention can rapidly increase the number of obtainable clinical reports. As a result, such reports of measurement of a specific gas which hitherto unclear to us may lead to large contribution to medical science, such as diagnosis of a new disease or identification thereof and interpretation of unidentified phenomena unpredictable now.

## Claims

1. An expired air examination device for clinical purpose comprising an expired air sample injecting part feeding to a column an expired air collected in an expired air collecting means, a carrier gas feeding part sending a carrier gas which feeds the expired air sample into the column, a detecting part including a detector which detects trace amounts of gas components contained in the expired air sample and separated in the column and outputs measurement signals, and an arithmetic processing part which processes the measurement signals, computes concentration of the aimed gas by use of a previously memorized working curve, and stores the computed concentration of the gas as a clinical examination data or outputs the computed concentration of the gas on an indicating device and/or a recording device, characterized by a small-sized detector of high sensitivity which ionizes the trace amounts by application of ultraviolet radiation and whose output signals corresponde to an amount of ionization of the aimed gas and by a carrier gas generating device which feeds a purified air serving as the carrier gas to the detecting part comprising a column and the detector which ionizes trace amounts of aimed gas component in gaseous phase to detect the aimed gas, which carrier gas generating device comprises an adsorbing filter, a compression pump for giving pressure to air passed the absorbing filter and a flow adjuster means.

2. An expired air examination device as set forth in claim 1, wherein the expired air collecting means comprise an expired air sucking part comprising an expired air collector in the form of mouthpiece or an expired air collecting mask and a collecting tube or a sampling probe mounting the expired air collector at utmost end, and the collecting tube or the sampling probe is provided circumferentially or in the inside with a heating element or made of a material having itself heat build-up, and the collecting tube or the sampling probe is sheathed on the outer periphery with a heat insulating material.

3. An expired air examination device for clinical purpose as set forth in claim 1, which device comprises a first adsorbing filter, a mixing tank which mixes air passed through the first adsorbing filter with a waste carrier gas from the detector, a second adsorbing filter which treats the air from the mixing tank, a compression pump and a flow adjuster means.

## Patentansprüche

1. Ausatemluftuntersuchungsvorrichtung für klinische Zwecke mit einem Ausatemluftprobeninjektionsteil, der eine in einem Ausatemluftsammelmittel gesammelte Ausatemluft einer Säule zuführt, einem Trägergaszuführteil, das ein Trägergas, das die Ausatemluft in die Säule eingibt, abgibt, einem Detektorteil mit einem Detektor, der Spuren von Gasbestandteilen, die in der Ausatemluft enthalten sind und in der Säule getrennt wurden, detektiert und Meßsignale ausgibt, und einem arithmetischen Rechenteil, das die Meßsignale verarbeitet, die Konzentration des Zielgases mittels einer zuvor eingespeicherten Arbeitskurve berechnet und die berechnete Konzentration des Gases als klinische Untersuchungsdaten abspeichert oder die berechnete Konzentration des Gases an einer Anzeigevorrichtung und/oder einer Aufzeichnungsvorrichtung ausgibt, gekennzeichnet durch einen kleinbauenden Detektor mit hoher Empfindlichkeit, der die Spuren durch Anwendung ultravioletter Strahlung ionisiert und dessen Ausgangssignal dem Ionisierungsgrad des Zielgases entspricht, und durch eine Trägergaserzeugungsvorrichtung, die gereinigte Luft als Trägergas dem Detektorteil mit der Säule und dem Detektor zuführt, welcher die Spuren der Zielgasbestandteile in gasförmiger Form zur Detektion des Zielgases ionisiert, welche Trägergaserzeugungsvorrichtung einen Adsorptionsfilter, eine Verdichtungspumpe zum Verdichten der durch das Adsorptionsfilter geleiteten Luft und ein Flußeinstellmittel aufweist.

2. Ausatemluftuntersuchungsvorrichtung nach Anspruch 1, bei der die Ausatemluftsammelmittel einen Ausatemluftansaugteil mit einem Ausatemluftsammler in Form eines Mundstückes oder einer Ausatemluftsammelmaske und eine Sammelröhre oder eine Sammelsonde, die am äußersten Ende des Ausatemluftsammlers befestigt ist, aufweist, wobei die Sammelröhre oder die Sammelsonde am Rand oder an der Innenseite ein Heizelement hat oder aus einem Material besteht, das selbst Wärme erzeugt, und wobei die Sammelröhre oder die Sammelsonde außen mit einem Wärmeisolationsmaterial abgeschirmt ist.

3. Ausatemluftuntersuchungsvorrichtung für klinische Zwecke nach Anspruch 1, die einen ersten Adsorptionsfilter, einen Mischtank, der durch den ersten Adsorptionsfilter geführte Luft mit verbrauchtem Trägergas aus dem Detektor mischt, einen zweiten Adsorptionsfilter, der Luft aus dem Mischtank behandelt, eine Verdichterpumpe und ein Flußeinstellmittel aufweist.

## Revendications

1. Dispositif d'examen d'air expiré destiné à un usage clinique comprenant une partie d'injection d'échantillon d'air expiré amenant à une colonne un air expiré recueilli dans un moyen de collecte d'air expiré, une partie d'alimentation de gaz vecteur émettant un gaz vecteur qui amène l'échantillon d'air expiré jusque dans la colonne, une partie de détection comprenant un détecteur qui détecte des quantités en traces des composants gazeux contenus dans l'échantillon d'air expiré et séparés dans la colonne et fournit en sortie des signaux de mesure, et une partie de traitement arithmétique qui traite les signaux de mesure, calcule la concentration du gaz recherché en utilisant une courbe de travail mémorisée au préalable et mémorise la concentration calculée du gaz sous forme de données d'examen clinique ou fournit en sortie la concentration calculée du gaz sur un dispositif d'indication et/ou un dispositif d'enregistrement, caractérisé par un détecteur de petite taille, de haute sensibilité qui ionise les quantités en traces par l'application d'un rayonnement ultraviolet et dont les signaux de sortie correspondent à une quantité d'ionisation du gaz recherché et par un dispositif de génération de gaz vecteur qui amène un air purifié servant de gaz vecteur à la partie de détection comprenant une colonne et au détecteur qui ionise des quantités en traces du composant gazeux recherché en phase gazeuse pour détecter le gaz recherché, lequel dispositif de génération de gaz vecteur comprend un filtre d'adsorption, une pompe de compression destinée à appliquer une pression à l'air qui a franchi le filtre d'absorption et un moyen de réglage de débit.

2. Dispositif d'examen d'air expiré selon la revendication 1, dans lequel le moyen de collecte d'air expiré comprend une partie d'aspiration d'air expiré comprenant un collecteur d'air expiré sous forme d'un embout buccal ou d'un masque de collecte d'air expiré, et un tube de collecte ou une sonde d'échantillonnage recevant le collecteur d'air expiré à l'extrémité finale, et le tube de collecte ou la sonde d'échantillonnage est muni à sa circonférence ou à l'intérieur d'un élément de chauffage ou est constitué d'un matériau présentant lui-même une accumulation de chaleur, et le tube de collecte ou la sonde d'échantillonnage est gainé sur la périphérie extérieure par un matériau thermiquement isolant.

3. Dispositif d'examen d'air expiré destiné à un usage clinique selon la revendication 1, lequel dispositif comprend un premier filtre d'adsorption, un réservoir de mélange qui mélange de l'air qui a franchi le premier filtre d'adsorption à un gaz vecteur utilisé provenant du détecteur, un second filtre d'adsorption qui traite l'air provenant du réservoir de mélange, une pompe de compression et un moyen de réglage de débit.
